Europäisches Patentamt

European Patent Office

Office européen des brevets

(11) Numéro de publication: **0 211 777**
**B1**

(12) **FASCICULE DE BREVET EUROPEEN**

(45) Date de publication du fascicule du brevet: 03.10.90

(51) Int. Cl.⁵: **G 01 N 33/548,** C 12 Q 1/68,
B 01 L 3/00

(21) Numéro de dépôt: **86420170.2**

(22) Date de dépôt: **26.06.86**

(54) Dispositif pour détecter sur une feuille de nitrocellulose la présence de complexes macromoléculaires, tels que antigènes/anticorps et procédé de mise en oeuvre.

(30) Priorité: 02.07.85 FR 8510271

(43) Date de publication de la demande:
25.02.87 Bulletin 87/09

(45) Mention de la délivrance du brevet:
03.10.90 Bulletin 90/40

(84) Etats contractants désignés:
AT BE CH DE FR GB IT LI LU NL SE

(56) Documents cités:
EP-A-0 118 735
EP-A-0 119 858
US-A-4 031 197
US-A-4 427 415

(73) Titulaire: Etablissement Public dit: CENTRE
NATIONAL DE LA RECHERCHE SCIENTIFIQUE
(CNRS)
15, Quai Anatole France
F-75007 Paris (FR)

(72) Inventeur: Alric, Monique
33 boulevard Claude Bernard
F-63000 Clermont-Ferrand (FR)
Inventeur: Renaud, Michel P.
4 rue du Château
F-63670 Le Cendre (FR)

(74) Mandataire: Laurent, Michel et al
Cabinet LAURENT et GUERRE B.P. 32
F-69131 Ecully Cedex (FR)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

L'invention concerne un dispositif pour détecter la présence sur une feuille type nitrocellulose de complexes macromoléculaires, tels que par exemple des complexes antigènes/anticorps; elle se rapporte également à un procédé pour la mise en oeuvre de ce dispositif.

Bien que dans la suite du texte, l'invention soit plus particulièrement décrite dans sa forme de réalisation à la détection des complexes antigènes/anticorps, elle n'est nullement limitée à ce mode de réalisation, puisque d'autres réactants, notamment d'autres protéines, ARN et ADN peuvent être également utilisés.

Pour la détection des complexes antigènes/anticorps sur une feuille type nitrocellulose, on fait généralement appel à une méthode dénommée "ELISA". On connait déjà un dispositif décrit dans le brevet US—A—4 031 197 mettant en oeuvre cette méthode constitué essentiellement par une plaque en matière plastique comportant une pluralité de puits parallèles en forme de U que l'on remplit individuellement avec chacun des réactants. Bien que cette méthode soit très largement répandue, ce dispositif, nécessite des puits témoins pour effectuer la comparaison, et enfin et surtout, nécessite un nombre important de manipulations et une quantité substantielle de réactants. Par "feuille type nitrocellulose", on désigne non seulement les feuilles de nitrate de cellulose d'emploi courant, mais également les feuilles équivalentes, telles que par exemple celles en acétate de cellulose.

Récemment, on a décrit et même commercialisé des appareils dont la plaque supérieure est constituée de puits sans fond disposés comme dans les plaques "ELISA". Cette plaque est déposée sur une feuille de nitrocellulose elle-même posée sur une deuxième plaque percée de trous en correspondance avec les premiers, mais de diamètre moindre, puis le vide est appliqué sur le fond par aspiration, l'ensemble étant maintenu serré pendant toute cette opération (voir par exemple brevet américain US—A—4 427 415 et brevet européen EP—A—0 118 735). Toutefois, cette méthode reste encore longue à metre en oeuvre et nécessite un personnel assez qualifié. En outre et surtout, cette méthode comporte deux inconvénients majeurs, à savoir l'impossibilité d'anlyser directement les composants du bruit de fond et la spécificité de certaines réponses.

Dans "Analytical Biochemistry" 138, 119—124 (1984), on a décrit un système constitué par:

un socle de fond en aluminium en forme de plaque destiné à recevoir sur le dessus une feuille de caoutchouc siliconé et une feuille de nitrocellulose;

une plaque dont l'envers présente une pluralité de canaux ou de rainures parallèles ouvertes seulement à leur extrémité (voir aussi brevet européen EP—A—0 119 858).

Ce dispositif d'analyse est applicable seulement à la détection des BLOTS, c'est-à-dire des macromolécules biologiques telles que des protéines, ADN ou ARN, transférées sur la feuille de nitrocellulose après une électrophorèse sur gel, et non pas la détection des DOTS, cette détection désignant ces mêmes macromolécules disposées directement sur la feuille de nitrocellulose. De plus, l'architecture même de ce dispositif entraîne la présence quasi-permanente de bulles d'air dans les canaux, ce qui d'une part gêne le remplissage de ces canaux et d'autre part et surtout, perturbe les réponses. Elle entraîne également la présence de fuites entre les canaux lorsque le temps d'accumulation dépasse quelques heures.

L'invention pallie ces inconvénients. Elle vise un dispositif de détection du type en question qui soit facile et économique à réaliser, commode et rapide à manipuler, même avec du personnel non qualifié, permette d'utiliser une faible quantité de matière, même non parfaitement purifiée, permette également de réaliser un grand nombre de combinaisons entre les deux réactants et enfin donne des réponses sans ambiguités et soit donc fiable.

Ce dispositif pour détecter sur une feuille de nitrate ou d'acétate de cellulose, la présence d'un complexe macromoléculaire, comprenant:

un fond destiné à recevoir ladite feuille de nitrate ou d'acétate de cellulose;

une plaque destinée à reposer sur ladite feuille de nitrate ou d'acétate de cellulose, appliquée sur le fond, comportant une pluralité de rainures parallèles;

des organes de serrage de la plaque rainurée sur le fond;

se caractérise:

en ce que le fond a la forme d'un boîtier dans lequel est ménagé un compartiment adapté pour recevoir ladite feuille de nitrate ou d'acétate de cellulose et ladite plaque;

en ce que la pluralité de rainures parallèles traversent ladite plaque sur toute son épaisseur;

et en ce que le dispositif comprend également une semelle intercalaire destinée à être disposée entre le fond du compartiment du boîtier et la feuille de nitrate ou d'acétate de cellulose, la face de cette semelle dirigée contre le fond du compartiment présentant des protubérances dont la hauteur décreoît depuis le centre jusque vers le bord de la semelle intercalaire.

En d'autres termes, l'invention consiste d'une part à ne plus faire appel à des rainures parallèles ouvertes seulement à leur extrémité, mais à des rainures parallèles ouvertes sur toute la longueur et sur toute l'épaisseur de la plaque, et d'autre part à intercaler entre le fond et la feuille de nitrate ou d'acétate de cellulose une semelle dont la structure particulière assure une meilleure étanchéité lorsque l'ensemble est en pression en facilite un léger glissement latéral de la plaque rainurée caractéristique. Ainsi, après croisement de telles plaques, on peut étudier aussi bien des DOTS que des BLOTS, et par voie de conséquence, on peut ainsi multiplier le nombre des combinaisons, ce que l'on ne savait pas faire économiquement jusqu'alors de manière rapide et fiable. Ainsi, grâce à cette simple disposition, on peut étendre considérablement le champ d'application et l'intérêt de ce dispositif.

Avantageusement en pratique:

on emboîte dans le fond, constitué d'une ou plusieurs pièces, la plaque rainurée dont le dessus est recouvert a son tour par un couvercle, l'ensemble: boîtier, plaque, couvercle est enserré par des organes de fermeture appropriés; ces organes de fermeture, tels que par exemple des pinces ou des vis doivent permettre de répartir la pression de manière uniforme sur toute la surface de la plaque rainurée être pratiques, simples à manipuler et ne pas être encombrants;

on dispose un joint entre la feuille de nitrate ou d'acétate de cellulose et le fond du boîtier, constitué par exemple par une feuille de papier humidifié ou par une membrane élastique; dans une première forme de réalisation, ce joint est constitué par un papier fortement absorbant humidifié; dans une seconde forme de réalisation avantageuse, la feuille de papier humidifié est disposée sur un empilement de feuilles de papier élémentaires sèches, ce qui permet l'adsorption rapide du premier réactant sur la feuille de nitrate ou d'acétate de cellulose; avantageusement, le joint est constitué d'une membrane souple élastique; de même, ce joint peut être constitué par un empilement de feuilles de papier humidifié recouvert d'une feuille étanche, type parafilm;

dans une autre configuration, le fond du boîtier comporte un compartiment pouvant être relié à une chambre à vide dans laquelle vient s'emboîter une autre plaque formant fond percée d'orifices traversant, disposée ou non en regard des rainures traversantes de la première plaque, une feuille de papier étant interposée entre cette deuxième plaque de fond et la feuille de nitrate ou d'acétate de cellulose qui est toujours au contact de la première plaque rainurée; en pratique, les orifices traversant de la deuxième plaque sont constitués par des trous ou des rainures de petites dimensions;

les rainures ouvertes parallèles d'une même plaque sont discontinues, mais de longueur égale;

le fond, le boîtier et la plaque rainurée sont en matière plastique moulée ou usinée;

l'ensemble comprend une série de plaques rainurées dont la direction des rainures est orthogonale d'une plaque par rapport à l'autre;

les rainures parallèles ouvertes ont une largeur comprise entre un et quatre millimètres; si cette largeur est inférieure à un millimètre, on risque de voir apparaître des phénomènes de capillarité sur les parois; en revanche, si cette largeur est supérieure à quatre millimètres, pour la même surface, on diminue inutilement le nombre de combinaisons réalisables, et on en assure en outre un serrage moins efficace; si la hauteur des rainures, donc de la plaque qui les comporte, n'est ni significative ni critique, en revanche, il importe que la longueur de ces rainures soit adaptée à l'utilisation désirée (BLOT ou DOT). Utilement, dans l'utilisation BLOT, la longueur de ces rainures correspond à celles du gel d'électrophorèse

grâce auxquels sont préparés les BLOTS, tel que par exemple un gel de polyacrylamide; enfin la distance entre deux rainures parallèles successives doit tenir compte des données de fabrication et d'utilisation pour éviter une rupture intempestive; économiquement, la plaque rainurée et moulée avec des dépouilles appropriés de l'ordre de un à trois degrés par exemple;

le dispositif comprend en outre une partie contigue au compartiment, et recevant un organe de fermeture constitué par un bouton de manoeuvre rotatif asymétrique, dont la périphérie forme came et porte appui sur le bord de la plaque rainurée pour bloquer celle-ci dans le compartiment.

L'invention concerne également un procédé pour la mise en oeuvre de ce dispositif.

Ce procédé pour détecter sur une feuille de nitrate ou d'acétate de cellulose, la présence de macromolécules biologiques et qui consiste:

à déposer une feuille de nitrate ou d'acétate de cellulose sur au moins une membrane formant joint disposée sur une première plaque de fond;

à recouvrir cette feuille d'une seconde plaque comportant une pluralité d'orifices;

après avoir serré lesdites plaques, à remplir les rainures ouvertes avec un premier réactant;

à laisser incuber ce premier réactant, sur ladite feuille de nitrate ou d'acétate de cellulose;

à vidanger et à rincer le contenu des rainures parallèles ouvertes;

après avoir retiré la deuxième plaque, à laisser incuber la feuille de nitrate ou d'acétate de cellulose pour saturer les sites aspécifiques;

à méanger dans la seconde plaque une pluralité de rainures parallèles;

à intercaler entre la membrane formant joint disposé sous la feuille de nitrate ou d'acétate de cellulose et le fond du boîtier une semelle dont la face dirigée vers le fond du boîtier présente des protubérances, dont la hauteur décroît du centre vers le bord;

à recouvrir à nouveau ladite feuille de nitrate ou d'acétate de cellulose incubée d'une autre plaque comportant également une pluralité de rainures parallèles la traversant sur toute son épaisseur et sur toute la longueur de ces rainures, mais dont la direction des rainures est orthogonale à la direction des rainures de la deuxième plaque de manière à croiser sur la feuille de nitrate ou d'acétate de cellulose les interactions provoquées par lesdites rainures;

après avoir à nouveau serré lesdites plaques à remplir ces rainures ouvertes d'un deuxième réactant; et

à laisser incuber, vidanger et rincer puis retirer cette troisième plaque et enfin à incuber la feuille de nitrate ou d'acétate de cellulose ainsi imprégnée.

Ce procédé est particulèrement adapté à la détection avec les systèmes CROSS DOTS.

Avantageusement en pratique:

les réactants sont formés par des solutions contenant un ou plusieurs réactants;

l'incubation s'effectue à la température désirée, notamment de 4 à 37°C, le temps d'incubation variant de quelques minutes à quelques heures;

la vidange s'effectue simplement soit en retournant l'ensemble fermé serré, soit par l'aspiration à l'aide d'une pipette ou d'une pompe à vide.

Si la seconde et la troisième plaques ont les mêmes dimensions extérieures, après démontage, après la première incubation, cette plaque est replacée après décalage à 90° d'angle sur la feuille de nitrate ou d'acétate de cellulose incubée afin d'obtenir le croisement sur cette feuille de nitrate ou d'acétate de cellulose des interactions provoquées par les rainures. Si la seconde et la troisième plaques rainurées sont rectangulaires, les rainures de la deuxième plaque sont disposées à angle droit par rapport à celle de la troisième plaque afin d'obtenir le décroisement.

La manière dont l'invention peut être réalisée et les avantages qui en découlent ressortiront mieux des exemples de réalisation qui suivent donnés à titre indicatif et non limitatif à l'aide des figures annexées.

La figure 1 est une vue en perspective sommaire éclatée d'un premier dispositif conforme à l'invention.

Les figures 2 et 3 montrent une représentation de la plaque rainurée caractéristique de l'invention respectivement vu de dessus (figure 2) et vu en coupe (figure 3).

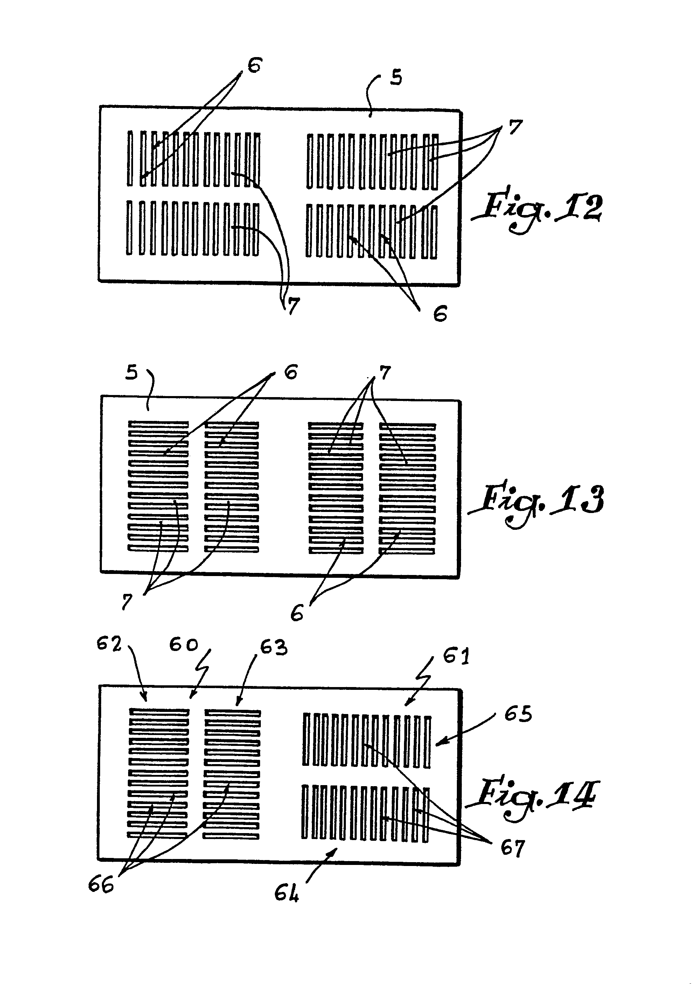

La figure 4 est une vue en coupe du dispositif de la figure 1 dans lequel tous les éléments ont été montrés écartés alors qu'en figure 5 ces mêmes éléments sont mis en place.

La figure 6 est une représentation sommaire destinée à expliquer le fonctionnement du dispositif selon l'invention.

Les figures 7 et 8 montrent une autre forme de réalisation de l'invention, respectivement vue de dessus (figure 7) et en coupe longitudinale (figure 8).

Les figures 9 à 14 montrent en vue de dessus différentes sortes de réalisation des plaques caractéristiques de l'invention.

Comme déjà dit, bien que l'utilisation décrite concerne la détection des complexes antigènes/anticorps, son mode de réalisation est simplement préféré.

Le dispositif selon l'invention comprend essentiellement trois pièces principales en matière plastique moulée, à savoir respectivement:

un boitier (1) dans lequel est ménagé un compartiment (2) sur le fond (3) duquel va reposer la feuille de papier (13) ou une membrane étanche élastique et la feuille de nitrocellulose (4);

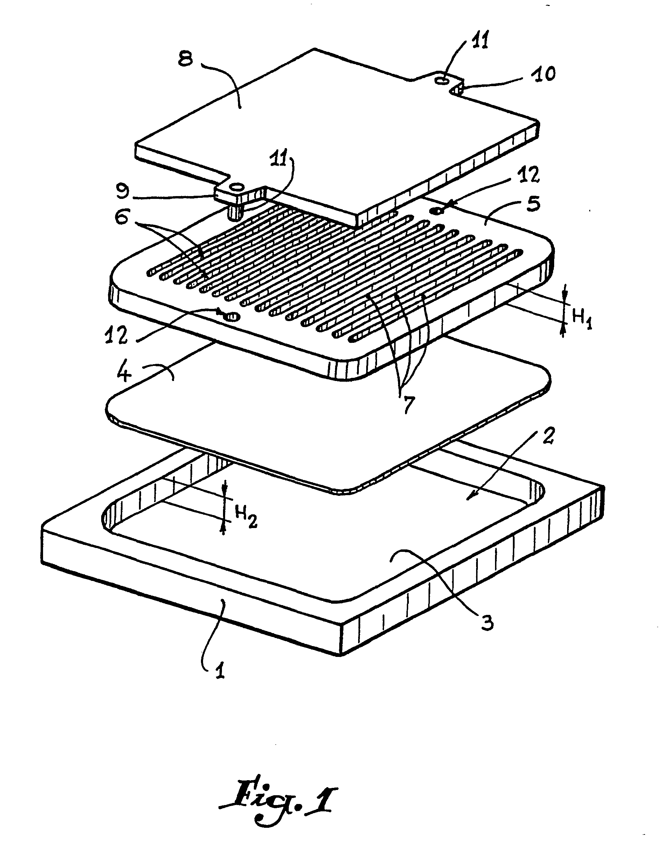

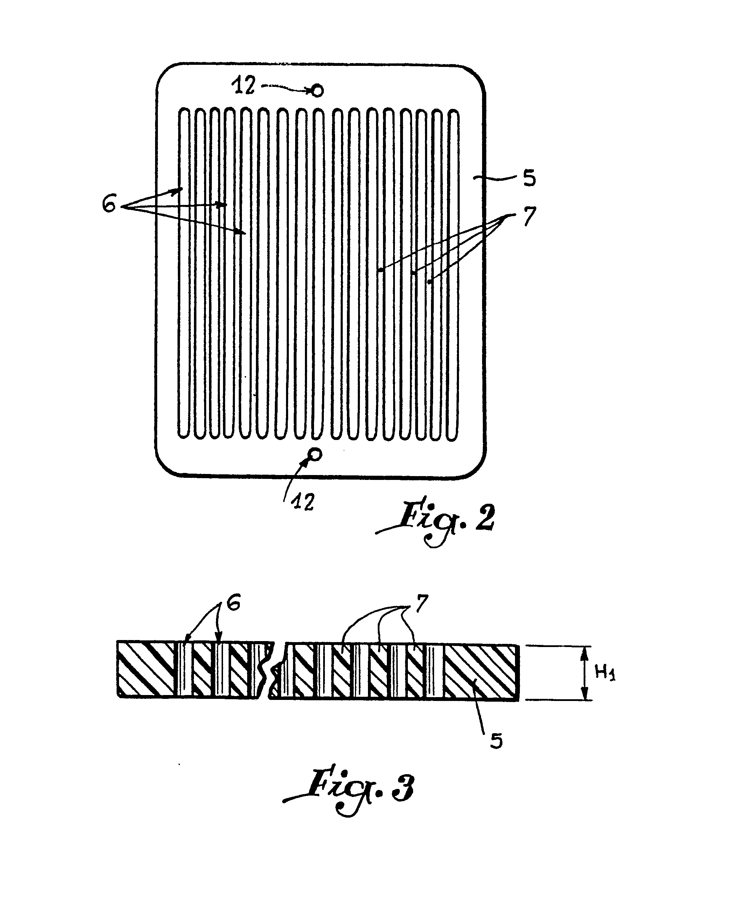

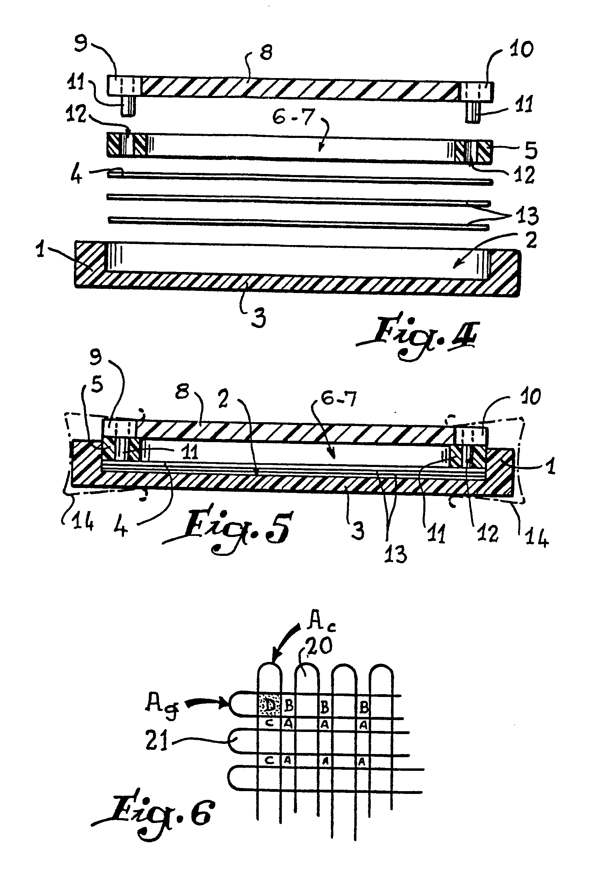

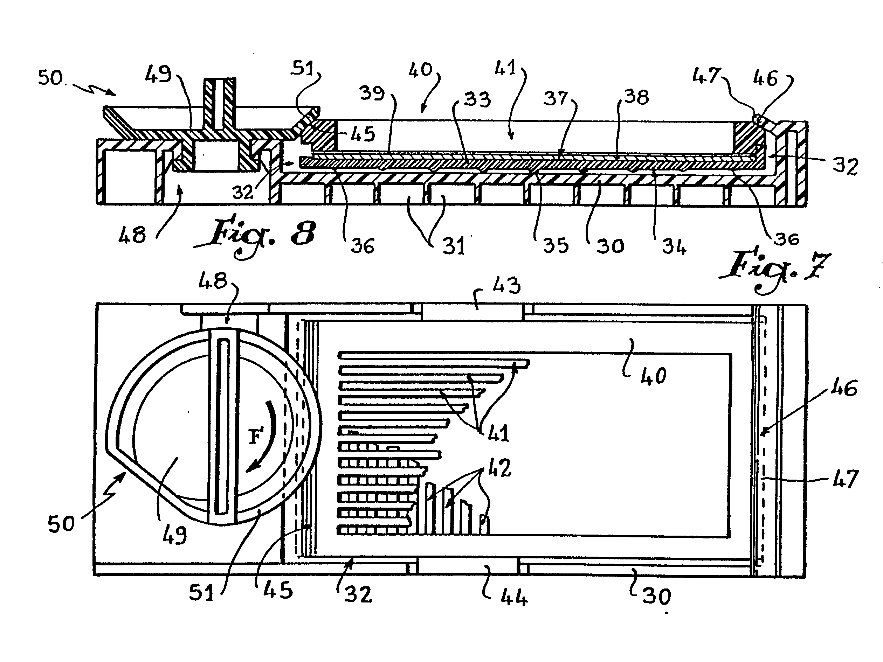

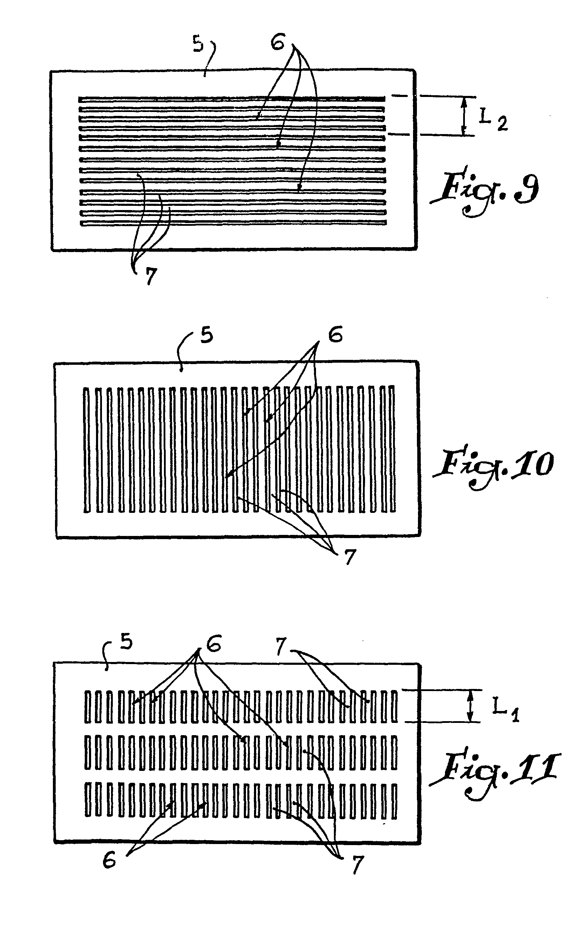

une première plaque (5) dans laquelle sont ménagées une pluralité de rainures parallèles traversantes (6), ouvertes sur toute la hauteur H1 de cette plaque (5) et sur toute la longueur de ces rainures (voir figures 2 et 3) séparées par des interpuits (7); la hauteur $H_1$ de cette deuxième plaque (5) est égale à la hauteur $H_2$ du compartiment (2) du boitier, alors que la longueur et la largeur de cette même deuxième plaque sont légèrement inférieures à celles du compartiment (2) et ce, afin de pouvoir être facilement retirée de ce compartiment (2);

un couvercle (8) plein qui recouvre au moins la surface rainurée de la plaque (5), comportant deux oreilles latérales (9) et (10) qui facilitent la préhension et sur la face envers desquelles sont montés des axes (11) destinés à venir s'emmancher dans des orifices (12) prévus à cet effet sur le dessus de la plaque (5);

l'ensemble est maintenu serré par des organes de fermeture connus, tels que des pinces (14) (voir figure 5) ou analogues.

Dans une version non représentée, plus particulièrement destiné à des professionnels, le fond du boitier est relié à une source de vide.

La mise en oeuvre de ce dispositif dans son application à la détection des complexes antigènes/anticorps s'effectue selon le procédé défini ci-dessus en prenant soin avantageusement de recouvrir le fond (3) du compartiment (2) d'un joint (13), tel qu'une feuille de papier fortement absorbante humidifiée ou une membrane élastique sur laquelle va donc reposer la feuille de nitrocellulose (4).

Ainsi, grâce au croisement des plaques rainurées caractéristiques (voir figure 6) et grâce à la grande capacité de combinaison, et de la possibilité d'analyser de manière comparative l'origine du bruit de fond, ce dispositif permet d'effectuer une analyse quantitative et qualitative de ces nombreux paramètres influençant la formation et l'analyse du complexe antigène/anticorps. D'après les résultats obtenus (voir figure 6), on peut ainsi définir quatre régions sur la feuille de nitrocellulose (4). Ces quatre régions sont schématisées par les références A, B, C et D et contribuent de manière complémentaire à estimer les différentes composantes du bruit de fond, donc à analyser sans ambiguïté la réponse antigène/anticorps. La région A correspond au bruit de fond que l'on rencontre en l'absence d'antigènes et d'anticorps puisque cette zone n'a nullement été affectée. La région B permet d'estimer le bruit de fond du à l'antigène alors que la région C permet d'estimer le bruit de fond du à l'anticorps. En l'absence de toute réaction spécifique antigènes/anticorps, l'intensité maximum due à l'aspécificité est donc la somme des intensités des régions B et C. Par comparaison, l'intensité de la réponse de la région D donne la spécificité de celle-ci.

Dans l'exemple de réalisation industrielle montré aux figures 7 et 8, l'ensemble en matière plastique moulée comprend essentiellement deux parties contiguës. La première partie se compose d'un socle (30) alvéolaire (31), analogue à (2), qui présente sur le dessus et à droite un compartiment (32) analogue à (3). Dans ce compartiment (32), vient se loger une semelle intercalaire (33) dont la face inférieure (34) présente des protubérances demi-sphériques respectivement (35, 36) venues de moulage, dont la hauteur décroit du centre (35) vers le bord (36). La face supérieure (37) de cette semelle intercalaire (33) comporte un logement dans lequel vient s'appliquer le joint caractéristique (38) analogue à (13) tel qu'une membrane élastique ou une feuille de papier humidifiée. Sur ce joint (38), vient alors s'appliquer la feuille de nitrocellulose (39) analogue à (4).

Comme précédemment, la plaque rainurée caractéristique (40) vient s'appuyer sur cet empilement (39, 38, 33). La plaque caractéristique (40) comporte comme déjà dit des rainures parallèles respectivement selon le cas (41, 42) ouvertes sur toute leur hauteur et sur toute leur longueur.

Les protubérances demi-sphériques progressives (35, 36) assurent une meilleure étanchéité lorsque l'ensemble est en pression et facilitent un léger glissement latéral de la plaque rainurée caractéristique (40).

Des encoches latérales (43, 44) facilitent la mise en place et le retrait des plaques rainurées (40) des le compartiment (32) grâce à des oreilles désignées par les mêmes références (43, 44) prévues à cet effet lors du moulage de la plaque caractéristique (40).

La plaque (40) présente à chacune de ses extrémités des plans inclinés respectivement (45) et (46) qui assurent le blocage en coopérant avec l'organe de fermeture désigné par la référence générale (50). Le plan incliné de droite (46) coopère avec une languette fixe (47) venue de moulage formant butée, solidaire du socle (30). En revanche, le plan incliné de gauche (45) coopère avec l'organe de fermeture (50) proprement dit caractéristique que de l'invention qui forme la seconde partie.

Cet organe de fermeture désigné par la référence générale (50) positionné à gauche de la plaque caractéristique (40) coulisse dans un chemin (48) prévu à cet effet dans le socle (30). L'organe de fermeture comprend un bouton de manoeuvre (49) asymétrique dont la périphérie relevée (51) forme came en prenant appui sur le plan incliné correspondant (45). Ainsi, en tournant le bouton (49) dans le sens indiqué par la flèche F, la lèvre relevée (51) s'appuie sur le plan incliné (45). Simultanément, la plaque caractéristique (40) comprime la semelle intercalaire (33) et glisse légèrement sur la feuille de nitrocellulose (39) sans la rayer.

Les traits partiels symbolisés sur la figure 7 représentent le croisement des rainures parallèles caractéristiques respectivement (41, 42) de deux plaques respectives (40, 40') qui, après croisement, permettent d'apprécier l'intensité des réponses.

Comme déjà dit, les figures 9 à 14 montrent différentes formes de réalisation des plaques (5) caractéristiques de l'invention, qui peuvent être croisées entre elles.

Les figures 9 à 10 correspondent sensiblement aux plaques (5, 5') illustrées aux figures 1, 2 et 3, destinées à être croisées lors de leur mise en oeuvre.

Les figures 11 et 12 illustrent une variante de la plaque (5) selon la figure 10 dans laquelle les rainures (6) sont de longueurs égales mais discontinues et sont regroupées par groupes de deux (figure 12) ou trois (figure 11). La longueur discontinue des rainures L1 est égale à l'intervalle d'un nombre entier L2 de rainures (6) de l'autre plaque. Cette disposition permet d'augmenter considérablement le nombre de combinaisons à tester en une seule expérience. Par exemple, si l'on croise les deux plaques montrées aux figures 11 et 12 avec la plaque illustrée à la figure 9, on obtient respectivement (90×3=270 combinaisons) et (52×6=312 combinaisons).

La figure 13 représente une alternative de la figure 9 destinée à coopérer avec les plaques selon les figures 10, 11 et 12.

La plaque illustrée à la figure 14 comporte deux séries respectivement (60, 61) de rainures orthogonales parallèles, rassemblées chacune en deux groupes (62 à 65) de deux sous-groupes (62, 63) et (64, 65). Ces rainures parallèles (66) et (67) analogues à (6) sont en nombres égaux et ont toutes des longueurs et des largeurs égales. Ainsi, une seule et même plaque selon la figure 14 peut être utilisée pour la mise en oeuvre pour une expérience CROSS-DOT. Il suffit simplement de faire pivoter la plaque de 180° d'une phase à l'autre. Il va de soi que de nombreuses autres combinaisons de groupes de rainures parallèles peuvent être réalisées sans sortir du cadre de la présente invention.

Le dispositif et le procédé selon l'invention comportent de nombreux avantages par rapport à ceux connus à ce jour. On peut citer:

en ce qui concerne l'appareil:

un coût réduit,

le fait que le boitier peut recevoir des plaques différentes, ce qui augmente ainsi les possibilités de combinaison,

l'absence de fuites, donc absence de contamination;

en ce qui concerne le procédé:

le fait d'être rapide et facile à manipuler avec un nombre d'opérations réduit et ce, même sans faire appel à du personnel qualifié,

la possibilité d'utiliser les réactants dans n'importe quel ordre,

une excellente reproductibilité, puisque tous les carreaux résultant du même croisement ont la même intensité; cette reproductibilité n'est limitée que par la qualité de la feuille type nitrocellulose utilisée,

l'élimination directe des faux positifs,

la possibilité d'obtenir une analyse directe de la réponse et ainsi de comparer des affinités,

une économie de la quantité de réactants (de 20 à 30 fois selon le nombre de rainures croisées),

enfin, par rapport aux deux procédés cités dans le préambule:

d'une part, le nombre de combinaisons premier et deuxième réactants en une seule expérience est très supérieur à celui des systèmes existants, puisqu'il est plus que doublé, voire même décuplé selon l'utilisation;

en outre et surtout, la possibilité de réaliser comme déjà dit une analyse directe de la contribution de chacun des bruits de fond et du signal spécifique de l'interaction des réactants, bref la possibilité d'estimer immédiatement et directement sans expérience supplémentaire si le deuxième réactant est spécifique, ce qui permet ainsi de porter un diagnostic.

On obtient ainsi un dispositif aux multiples possibilités pour la détection de réactants macromoléculaires très complexes, notamment des macromolécules biologiques, tels que des protéines (complexes antigènes/anticorps, ADN/ARN..) en toute combinaison. Ainsi, ce dispositif et cette méthode sont particulièrement adaptés au diagnostic médical, agricole, vétérinaire, biochimique par des professionnels qualifiés ou même par des utilisateurs non spécialisés.

## Revendications

1. Dispositif pour détecter la présence de complexes macromoléculaires sur une feuille de nitrate ou d'acétate de cellulose, comprenant:

un fond (1) destiné à recevoir ladite feuille de nitrate ou d'acétate de cellulose (4, 39),

un plaque (5, 40) destinée à reposer sur ladite feuille de nitrate ou d'acétate de cellulose (4, 39), appliquée sur le fond (1), comportant une pluralité de rainures parallèles,

des organes de serrage (14, 50) de la plaque rainurée (5, 40) sur le fond (1, 30), caractérisé:

en ce que le fond (1) a la forme d'un boîtier (30) dans lequel est ménagé un compartiment (32) adapté pour recevoir ladite feuille de nitrate ou d'acétate de cellulose (39) et ladite plaque (40);

—en ce que la pluralité de rainures parallèles (41, 42) traversent ladite plaque (5, 40) sur toute son épaisseur ;

et en ce que le dispositif comprend également une semelle intercalaire (33) destinée à être disposée entre le fond (30) du compartiment (32) du boîtier et la feuille de nitrate ou d'acétate de cellulose (39), la face (34) de cette semelle (33) dirigée contre le fond du compartiment (30, 32) présentant des protubérances (35, 36) dont la hauteur décroît depuis le centre (35) jusque vers le bord (36) de la semelle intercalaire (33).

2. Dispositif selon la revendication 1, caractérisé en ce qu'il comporte une membrane (38) formant joint, destinée à être intercalée entre le fond (32) du boîtier (30) et la feuille de nitrate ou d'acétate de cellulose (39).

3. Dispositif selon l'une des revendications 1 et 2 caractérisé en ce que les rainures ouvertes parallèles (6) d'une même plaque (5, 40) sont discontinues, mais de longueur égale.

4. Dispositif selon l'une des revendications 1 à 3, caractérisé en ce que le fond (1), le boîtier (30) et la plaque rainurée (5, 40) sont en matière plastique moulée.

5. Dispositif selon l'une des revendications 1 à 4, caractérisé en ce que l'ensemble comprend une série de plaques rainurées (5, 40) dont la direction des rainures (6, 41, 42) est orthogonale d'une plaque par rapport à l'autre, et en ce que les rainures parallèles (6, 41, 42) ouvertes ont une largeur comprise entre un et quatre millimètres.

6. Dispositif selon l'une des revendications 1 à 5, caractérisé en ce qu'il comprend en outre une partie contigüe au compartiment (32) recevant un organe de fermeture (50) constitué par un bouton de manoeuvre (49) rotatif asymétrique, dont la périphérie (51) forme came et prend appui sur le bord (45) de la plaque rainurée (40) pour bloquer celle-ci dans le compartiment (32).

7. Procédé pour la détection sur une feuille de nitrate ou d'acétate de cellulose (4, 39) de la présence de macromolécules biologiques, qui consiste:

à déposer une feuille de nitrate ou d'acétate de cellulose (4, 39) sur au moins une membrane formant joint (13, 38) disposée sur une première plaque (1, 10) de fond;

à recouvrir cette feuille (4, 39) d'une seconde plaque (5, 40) comportant une pluralité d'orifices;

après avoir serré (14, 50) lesdites plaques (1, 5), à remplir les rainures (6) ouvertes avec un premier réactant;

à laisser incuber ce premier réactant sur ladite feuille de nitrate ou d'acétate de cellulose (4, 39);

à vidanger et à rincer le contenu des rainures parallèles ouvertes (6, 41, 42);

après avoir retiré la deuxième plaque (50), à laisser incuber la feuille de nitrate ou d'acétate de cellulose (4, 39) pour saturer les sites aspécifiques;

à ménager dans la seconde plaque (5, 40) une pluralité de rainures parallèles;

à intercaler entre la membrane formant joint (38) disposé sous la feuille de nitrate ou d'acétate de cellulose (4, 39) et le fond (30) du boîtier, une semelle (33) dont la face (34) dirigée vers le fond (30) du boîtier présente des protubérances (35, 36) dont la hauteur décroît du centre (35) vers le bord (36);

à recouvrir à nouveau ladite feuille de nitrate ou d'acétate de cellulose (4, 39) incubée d'une autre plaque (5, 40) comportant également une pluralité de rainures parallèles la traversant sur toute son épaisseur et sur toute la longueur de ces rainures, mais dont la direction des rainures (6, 42) est orthogonale à la direction des rainures (6, 41) de la deuxième plaque (5, 40) de manière à croiser sur la feuille de nitrate ou d'acétate de cellulose (4, 39) les interactions provoquées par les rainures;

puis, après avoir à nouveau serré (14, 50) lesdites plaques (1, 5, 40) à remplir ces rainures ouvertes d'un deuxième réactant; et

à laisser incuber, vidanger et rincer, puis retirer cette troisième plaque (5, 40) et enfin à incuber la feuille de nitrate ou d'acétate de cellulose (4, 39) ainsi imprégnée.

## Patentansprüche

1. Vorrichtung zum Ermitteln des Vorhandenseins von makromolekularen Komplexen auf einem Blatt aus Zellulosenitrat oder Zelluloseacetat,

mit einem Boden (1) zum Aufnehmen des Blattes (4, 39) aus Zellulosenitrat oder Zelluloseacetat,

mit einer, eine Vielzahl an paralleln Schlitzen

aufweisenden Platte (5, 40), die dazu vorgesehen ist, auf dem, auf den Boden (1) gelegten Blatt (4, 39) aus Zellulosenitrat oder Zelluloseacetat zu ruhen,

sowie mit Organen (14, 50) zum Schließen der geschlitzen Platte (5, 40) auf dem Boden (1, 30), dadurch gekennzeichnet,

daß der Boden (1) die Form eines Gehäuses (30) aufweist, in dem ein Fach (32) ausgespart ist, das dazu vorgesehen ist, das Blatt (39) aus Zellulosenitrat oder Zelluloseacetat und die Platte (40) aufzunehmen;

daß die Vielzahl an parallelen Schlitzen (41, 42) durch die gesamte Höhe der Platte (5, 40) reichen;

und daß die Vorrichtung eine Zwischenplatte (33) aufweist, die dazu vorgesehen ist, zwischen den Boden (30) des Faches (32) des Gehäuses und das Blatt (39) aus Zellulosenitrat oder Zelluloseacetat angeordnet zu werden, wobei die Seite (34) der Zwischenplatte (33), die dem Boden des Faches (30, 32) zugewandt ist, Vorsprünge (35, 36) aufweist, deren Höhe von der Mitte (34) der Zwischenplatte (33) aus bis zu deren Rand (36) abnimmt.

2. Vorrichtung nach Anspruch 1, dadurch gekennzeichnet, daß eine eine Verbindungsstelle schaffende Membran (38) vorgesehen ist, die dazu dient, zwischen den Boden (32) des Gehäuses (30) und dem Blatt (39) aus Zellulosenitrat oder Zelluloseacetat gelegt zu werden.

3. Vorrichtung nach einem der Ansprüche 1 oder 2, dadurch gekennzeichnet, daß die parallelen, offenen Schlitze (6) ein und derselben Platte (5, 40) unterbrochen sind, jedoch jeweils dieselbe Länge aufweisen.

4. Vorrichtung nach einem der Ansprüche 1 bis 3, dadurch gekennzeichnet, daß der Boden (1), das Gehäuse (30) und die geschlitzte Platte (5, 40) aus geformtem Kunststoffmaterial bestehen.

5. Vorrichtung nach einem der Ansprüche 1 bis 4, dadurch gekennzeichnet, daß der Zusammenbau eine Reihe an geschlitzten Platten (5, 40) aufweist, bei denen die Ausrichtung der Schlitze (6, 41, 42) von einer Platte zur andern rechtwinklig zueinander verlaufend ist, und daß die parallelen, offenen Schlitze (6, 41, 42) eine Breite zwischen einem und vier Millimeter aufweisen.

6. Vorrichtung nach einem der Ansprüche 1 bis 5, dadurch gekennzeichnet, daß sie darüberhinaus einem zum Fach (32) benachbarten Bereich aufweist, der ein Verschlußorgan (50) aufnimmt, das durch einen asymmetrischen, drehbaren Betätigungsknopf (49) gebildet wird, dessen äußerer Rand (51) eine Nocke bildet, und der auf den Rand (45) der geschlitzten Platte (40) angelegt werden kann, um diese im Fach (32) zu verriegeln.

7. Verfahren zum Ermitteln des Vorhandenseins von biologischen Makromolekülen auf einem Blatt (4, 39) aus Zellulosenitrat oder Zelluloseacetat, mit den Schritten

Auflegen eines Blattes (4, 39) aus Zellulosenitrat oder Zelluloseacetat auf zumindest eine, eine Verbindungsstelle (13, 38) bildende Membran, die auf einer ersten Bodenplatte (1, 10) angeordnet ist;

Bedecken dieses Blattes (4, 39) mit einer zweiten Platte (5, 40), die eine Vielzahl an Öffnungen aufweist;

Füllen der offenen Schlitze (6) mit einem ersten Reagenz, nachdem die Platten (1, 5) verriegelt (14, 50) wurden;

Inkubieren des ersten Reagenz auf dem Blatt (4, 39) aus Zellulosenitrat oder Zelluloseacetat;

Entleeren und Ausspülen des Inneren der offenen, parallelen Schlitze (6, 41, 42);

nach Abnehmen der zweiten Platte (50), Inkubieren des Blattes (4, 39) aus Zellulosenitrat oder Zelluloseacetat zur Sättingung der aspezifischen Bereiche;

Aussparen einer Vielzahl an parallelen Schlitzen in der zweiten Platte (5, 40);

Einlegen einer Zwischenplatte (33) zwischen die eine Verbindungsstelle (38) bildenden Membran, die unter dem Blatt (4, 39) aus Zellulosenitrat oder Zelluloseacetat liegt und dem Boden (30) des Gehäuses, wobei die Seite (34) der Zwischenplatte (33), die dem Boden (30) des Gehäuses zugewandt ist, Vorsprünge (35, 36) aufweist, deren Höhe von der Mitte (35) zum Rand (36) abnimmt;

erneutes Bedecken des inkubierten Blattes (4, 39) aus Zellulosenitrat oder Zelluloseacetat mit einer anderen Platte (5, 40), die gleichermaßen eine Vielzahl an parallelen Schlitzen aufweist, die über deren gesamte Dicke und über die gesamte Länge deren Schlitze reichen, wobei jedoch die Richtung der Schlitze (6, 42) rechtwinklig zu der Richtung der Schlitze (6, 41) der zweiten Platte (5, 40) verläuft, und zwar derart, daß die durch die Schlitze hervorgerufenen Wechselwirkungen auf dem Blatt (4, 39) aus Zellulosenitrat oder Zelluloseacetat gekreuzt werden;

anschließend, nachdem die Platten (1, 5, 40) erneut verriegelt (14, 50) wurden, Füllen der offenen Schlitze mit einem zweiten Reagenz; und

Inkubieren, Entleeren und Spülen sowie Abnehmen der dritten Platte (5, 40), und abschließend Inkubieren des derart imprägnierten Blattes (4, 39) aus Zellulosenitrat oder Zelluloseacetat.

**Claims**

1. Device for detecting on a nitrocellulose-type sheet or on a sheet of cellulose acetate the presence of macromolecular complexes, comprising:

a bottom (1) adapted to receive said nitrocellulose sheet or said sheet of cellulose acetate (4, 39);

a plate (5, 40) adapted to rest on said nitrocellulose sheet or said sheet of cellulose acetate (4, 39) applied on the bottom (1), comprising a plurality of parallel grooves;

closure members (14, 50) for clamping the grooved plate (5, 40) with the bottom (1); characterized:

in that the bottom (1) has the form of a box (30) in which is formed a compartment (32) adapted to receive said nitrocellulose sheet or said sheet of cellulose acetate (39) and said plate (40);

in that the plurality of parallel grooves (41, 42) pass through said plate (5, 40) over the whole of its thickness;

and in that the device, also comprises an interposed sole (33) intended to be disposed between the bottom (30) of the compartment (32) of the box and the nitrocellulose sheet or the sheet of cellulose acetate (39), the surface (34) of said sole (33) directed towards the bottom of the compartment (30, 32) presenting protuberances (35, 36) whose height decreases from the center (35) towards the edge (36) of the interposed sole.

2. Device according to claim 1, characterized in that it comprises a membrane (38) forming seal, intended to be interposed between the bottom (32) of the box (30) and the nitrocellulose sheet or the sheet of cellulose acetate (39).

3. Device according to one of claims 1 and 2, characterized in that the parallel open grooves (6) of a same plate (5, 40) are discontinuous, but of same length.

4. Device according to one of claims 1 to 3, characterized in that the bottom (1), the box (30) and the grooved plate (5, 40) are made of moulded plastic material.

5. Device according to one of claims 1 to 4, characterized in that the assembly comprises a range of grooved plates (5, 40), of which the direction of the grooves (6, 41, 42) is at right angles from one plate to another, and in that the parallel open grooves (6, 41, 42) have a width included between 1 and 4 millimeters.

6. Device according to one of claims 1 to 5, characterized in that it also comprises a part adjacent to the compartment (32) receiving a closure member (50) constituted by a rotating asymmetrical manoeuvring knob (49), whose periphery (51) forms a cam abutting on the edge (45) of the grooved plate (40) in order to lock it in the compartment (32).

7. Process for detecting on a nitrocellulose-type sheet or on a sheet of cellulose acetate (4, 39) the presence of biological macromolecules, that consists:

in depositing a nitrocellulose-type sheet or a sheet of cellulose acetate (4, 39) on at least one membrane forming seal (13, 38) disposed on a first bottom plate (1, 10);

in covering this sheet (4, 39) with a second plate (5, 40) comprising a plurality of openings;

after having clamped (14, 50) said plates (1, 5) together, in filling these open grooves (6) with a first reactant;

in leaving the first reactant to incubate on said nitrocellulose sheet or said sheet of cellulose acetate (4, 39);

in draining and rinsing the contents of the parallel open grooves (6, 41, 42);

after having removed the second plate (50), in leaving the nitrocellulose sheet or the sheet of cellulose acetate (4, 39) to incubate, in order to saturate the aspecific sites;

in providing in the second plate (5, 40) a plurality of parallel grooves;

in interposing between the membrane forming seal (38) disposed under the nitrocellulose sheet or the sheet of cellulose acetate (4, 39) and the bottom (30) of the box a sole (33), whose face (34) directed towards the bottom (30) of the box is presenting protuberances (35, 36), whose height decreases from the center (35) to the edge (36);

in again covering the nitrocellulose sheet or the sheet of cellulose acetate (4, 39) incubated with another plate (5, 40) also comprising a plurality of parallel grooves traversing this plate over the whole of its thickness and over the whole width of these grooves, but of which the direction of the grooves (6, 42) is at right angles to the direction of the grooves (6, 41) of the second plate (5, 40), so as to cross on the nitrocellulose sheet or the sheet of cellulose acetate (4, 39) the interactions provoked by the grooves;

then, after having again clamped (14, 50) said plates (1, 5, 40) together, in filling these open grooves with a second reactant;

in leaving to incubate, draining and rinsing then removing this third plate (5, 40);

and, finally, in incubating, the nitrocellulose sheet or the sheet of cellulose acetate (4, 39) thus impregnated.

*Fig.1*

1

Fig. 2

Fig. 3

Fig. 4

Fig. 5

Fig. 6

Fig. 8

Fig. 7

EP 0 211 777 B1

Fig. 9

Fig. 10

Fig. 11

5

*Fig.12*

*Fig.13*

*Fig.14*